(19) Europäisches Patentamt
European Patent Office
Office européen des brevets

(11) Veröffentlichungsnummer: **0 478 974 B1**

# (12) EUROPÄISCHE PATENTSCHRIFT

(45) Veröffentlichungstag der Patentschrift: **27.12.95**

(21) Anmeldenummer: **91114904.5**

(22) Anmeldetag: **04.09.91**

(51) Int. Cl.6: **C07D 333/36**, C07D 333/42, C07D 275/03, C07D 277/42, C07D 285/08, C07D 285/135, A01N 43/10, A01N 43/78, A01N 43/80, A01N 43/84

## (54) N-Heteroaryl-2-nitroaniline

(30) Priorität: **20.09.90 DE 4029771**

(43) Veröffentlichungstag der Anmeldung: **08.04.92 Patentblatt 92/15**

(45) Bekanntmachung des Hinweises auf die Patenterteilung: **27.12.95 Patentblatt 95/52**

(84) Benannte Vertragsstaaten: **BE CH DE ES FR GB IT LI NL**

(56) Entgegenhaltungen:
**EP-A- 0 135 472**
**EP-A- 0 328 954**
**EP-A- 0 406 700**
**GB-A- 1 533 236**
**US-A- 3 594 394**

**PATENT ABSTRACTS OF JAPAN vol. 13, no. 472 (C-647)(3820) 25 Oktober 1989, & JP-A-1 186849 (SUMITOMO CHEM. CO. LTD.) 26 Juli 1989,**

(73) Patentinhaber: **BASF Aktiengesellschaft**
**Carl-Bosch-Strasse 38**
**D-67063 Ludwigshafen (DE)**

(72) Erfinder: **Schubert, Juergen, Dr.**
**Am oberen Luisenpark 2**
**W-6800 Mannheim 1 (DE)**
Erfinder: **Kober, Reiner, Dr.**
**Im Schlittweg 20**
**W-6701 Fussgoenheim (DE)**
Erfinder: **Hamprecht, Gerhard, Dr.**
**Rote-Turm-Strasse 28**
**W-6940 Weinheim (DE)**
Erfinder: **Sauter, Hubert, Dr.**
**Neckarpromenade 20**
**W-6800 Mannheim 1 (DE)**
Erfinder: **Kardorff, Uwe, Dr.**
**D 3,4**
**W-6800 Mannheim 1 (DE)**
Erfinder: **Kuenast, Christoph, Dr.**
**Salierstrasse 2**
**W-6701 Otterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, vol. 78, no. 19, 14 Mai 1973 Columbus, Ohio, USA & FR-A-2130420 [Imperial chemical Industries], 8 Dezember 1972 Seite 127; Spalte 2; ref. no. 120275A

CHEMICAL ABSTRACTS, vol. 83, no. 11, 15 September 1975 Columbus, Ohio, USA Schaefer G.et al.: "2-Anilino-1,3,4-thiadiazoles,inhibitors of oxidative and photosynthetic phosphorylation." & Z.Naturforsch.,C:Biosci.1975,30c,No.3-4,183-9 Seite 109; Spalte 2; ref. no. 91476N

Revue Roumaine de Chimie vol. 14, no. 10, Oktober 1969, Bucarest Seiten 1285 - 1294; I.Simiti et al.: "Contribution à l'étude de quelques Hétérocycles.XII. L'Halogénation et la Nitration de l'anilino-2-méthyl-5-thiodiazole-1,3,4"

## Beschreibung

Die vorliegende Erfindung betrifft neue N-Heteroaryl-2-nitroaniline der allgemeinen Formel Ia und Ib

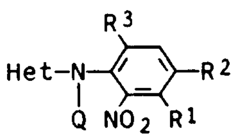

Ia

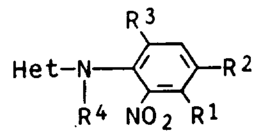

Ib

in der die Variablen die folgende Bedeutung haben:

- $R^1$ Wasserstoff, Halogen oder $C_1$-$C_4$-Alkoxy;
- $R^2$ Nitro, Cyano, Halogen, partiell oder vollständig halogeniertes $C_1$-$C_4$-Alkyl;
- $R^3$ Nitro, Halogen, $C_1$-$C_6$-Alkyl, partiell oder vollständig halogeniertes $C_1$-$C_4$-Alkyl;
- $R^4$ -CO-$R^5$, -CO-O$R^5$ oder -$SO_2R_5$ mit
  $R^5$, $C_1$-$C_4$-Alkyl, Phenyl oder Napththyl, die beide noch bis zu 3 Halogenatome und/oder $C_1$-$C_4$-Alkylgruppen tragen können;
- Q Wasserstoff, ein Alkalimetall- oder Erdalkalimetallion, ein Ammoniumion, dessen Stickstoffatom bis zu vier $C_1$-$C_4$-Alkyl-, Hydroxy-$C_1$-$C_4$-alkyl, Phenyl- und/oder Benzylsubstituenten tragen kann, ein Phosphonium-, Sulfonium- oder Sulfoxoniumion oder das Äquivalent eines Übergangsmetallkations;
- Het einen Thienyl-, Thiazolyl-, Isothiazolyl- oder Thiadiazolyl-Rest, der über ein Kohlenstoffatom des Ringes mit dem Grundkörper verknüpft ist, an den ein nichtaromatischer $C_5$-$C_8$-Ring anelliert sein kann und/oder dessen Grundkörper zusätzlich an jedem weiteren Kohlenstoffatom noch einen der folgenden Substituenten tragen kann: Cyano, Thiocyanato, Nitro, Halogen, $C_1$-$C_4$-Alkyl, partiell oder vollständig halogeniertes $C_1$-$C_4$-Alkyl, $C_3$-$C_{10}$-Cycloalkyl, $C_1$-$C_4$-Alkoxy, $C_1$-$C_4$-Alkylthio, Phenyl, Naphthyl, Benzyl oder Thienyl, wobei der Aromat jeweils bis zu drei Halogenatome und/oder $C_1$-$C_4$-Alkylgruppen tragen kann; CO-$R^5$, CO-O$R^5$, N$R^6R^7$, wobei $R^6$ und $R^7$ $C_1$-$C_4$-Alkyl, $C_2$-$C_4$-Alkenyl oder $C_2$-$C_4$-Alkinyl bedeuten,

ausgenommen diejenigen Verbindungen Ia, bei denen $R^1$ Wasserstoff, $R^2$ und/oder $R^3$ Nitro und Het einen durch Trifluormethyl oder tert.-Butyl substituierten 1,3,4-Thiadiazol-2-yl-Rest bedeuten.

Außerdem betrifft die Erfindung ein Verfahren zur Herstellung dieser Verbindungen, ihre Verwendung und die Verwendung weiterer Verbindungen vom Typ Ia als Mittel zur Bekämpfung von Insekten, Spinnentieren und Nematoden sowie Mittel, welche alle diese Verbindungen als wirksame Substanzen enthalten.

Aus den Druckschriften GB-A 1 533 236, DE-A 2 352 403, FR-A (Medicament) 7699, Chem. Ber. 95, 2511 (1962), Org. Mass Spectrom. 14(3), 171, (1979) und Revue Roumaine de Chimie 14, 1285 (1969) sind N-Heteroaryl-2-nitroaniline vom Typ der Verbindungen I bekannt, die sich jedoch durch ein anderes Substitutionsmuster am Phenylkern von den erfindungsgemäßen Verbindungen unterscheiden. Sie dienen als Zwischenprodukte oder im Falle der beiden französischen Druckschriften als pharmazeutische Wirkstoffe.

Des weiteren sind aus der Zeitschrift für Naturforschung 30c, 183 (1975) N-(1,3,4-Thiadiazol-2-yl)-2-nitroaniline bekannt, die in 2-, 4- oder 6-Stellung des Anilingrundkörpers zwei Nitrogruppen und einen weiteren Substituenten oder drei Nitrogruppen tragen können. Sie wirken im Pflanzenreich als Entkoppler und als Hemmstoffe des photosynthetischen Elektronentransportes.

Eine insektizide Wirkung wird in den genannten Druckschriften nicht angegeben.

In der EP-A 31 257 werden insektizid wirksame N-Pyridylaniline mit einem im Vergleich zu den Verbindungen I ähnlichen Substitutionsmuster am Anilin-Grundkörper beschrieben. Weiter sind aus JP-A 1 186 849 fungizid, mikrobizid, insektizid und herbizid wirksame Diphenylamin-Derivate bekannt. Die Wirkung dieser bekannten Pestizide auf die Schädlinge sowie die Dauer ihrer Wirkung vermag jedoch nur bedingt zu befriedigen. Daher lagen der Erfindung neue insektizid wirksame Substanzen als Aufgabe zugrunde, mit denen sich die Schädlinge besser als bisher bekämpfen lassen.

Demgemäß wurden die eingangs definierten N-Heteroaryl-2-nitroaniline der allgemeinen Formeln Ia und Ib gefunden.

Im einzelnen haben die Substituenten in den erfindungsgemäßen Verbindungen Ia bzw. Ib die folgende Bedeutung:

$R^1$

- Wasserstoff;
- Halogen wie Fluor, Chlor, Brom und Jod, insbesondere Fluor und Chlor;
- unverzweigtes oder verzweigtes $C_1$-$C_4$-Alkoxy wie Methoxy, Ethoxy, Isopropoxy und tert.-Butoxy, bevorzugt Methoxy und Ethoxy;

$R^2$

- Nitro oder Cyano;
- Halogen wie Fluor, Chlor, Brom und Jod, insbesondere Chlor;
- partiell oder vollständig halogeniertes $C_1$-$C_4$-Alkyl, insbesondere $C_1$-$C_2$-Alkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;

$R^3$

- Nitro;
- Halogen wie Fluor, Chlor, Brom und Jod, insbesondere Chlor;
- unverzweigtes oder verzweigtes $C_1$-$C_4$-Alkyl wie Methyl, Ethyl, Propyl, 1-Methylethyl, 1,1-Dimethylethyl, 1-Methyl-propyl, 2-Methylpropyl, n-Butyl oder tert.-Butyl;

$R^4$

- $-CO-OR^5$ oder $-SO_2-R^5$, mit

$R^5$

- unverzweigtes oder verzweigtes $C_1$-$C_4$-Alkyl wie Methyl, Ethyl, Propyl, 1-Methylethyl, 1,1-Dimethylethyl, 1-Methylpropyl, 2-Methylpropyl, n-Butyl oder tert.-Butyl;
- Phenyl oder Naphthyl, die beide 1 bis 3 Halogenatome, bevorzugt Fluor und Chlor und/oder $C_1$-$C_4$-Alkylgruppen, bevorzugt Methyl und Ethyl, tragen können, insbesondere Phenyl, 4-Fluorphenyl, 4-Chlorphenyl, 4-Bromphenyl, 2,4-Dichlorphenyl, 2-Methylphenyl, 3-Methylphenyl, 4-Methylphenyl, 2-Nitrophenyl, 4-Nitrophenyl, 1-Naphthyl, 2-Naphthyl und 4-Chlornaphth-1-yl;

Besonders bevorzugt werden $C_1$-$C_4$-Alkylcarbonyl wie Methylcarbonyl, Ethylcarbonyl, Propylcarbonyl und tert.-Butylcarbonyl, $C_1$-$C_4$-Alkoxycarbonyl wie Methoxycarbonyl, Ethoxycarbonyl, Propyloxycarbonyl, 1-Methylethoxycarbonyl, Butyloxycarbonyl, 1-Methyl-propyloxycarbonyl, 2-Methylpropyloxycarbonyl und 1,1-Dimethylethoxycarbonyl und $C_1$-$C_4$-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl und 1,1-Dimethylethylsulfonyl.

Q

- Wasserstoff;
- ein Alkalimetall- oder Erdalkalimetallion wie Natrium, Kalium, Calcium, Magnesium, Barium;
- ein Ammoniumion, dessen Stickstoffatom bis zu vier $C_1$-$C_4$-Alkyl-, Hydroxy-$C_1$-$C_4$-alkyl-, Phenyl- und/oder Benzylsubstituenten tragen kann, insbesondere ein Diisopropylammonium-, Tetramethylammonium-, Tetrabutylammonium-, Trimethylbenzylammonium- und Trimethyl-(2-hydroxyethyl)-ammoniumion;
- ein Phosphoniumion;
- ein Sulfoniumion, insbesondere ein Trialkylsulfoniumion oder ein Sulfoxoniumion;
- ein Äquivalent eines Übergangsmetallkations, insbesondere Mangan, Eisen, Kupfer und Zink;

Het: einen Thienyl-, Thiazolyl-, Isothiazolyl-, 1,2,4-Thiadiazolyl- oder 1,3,4-Thiadiazolyl-Rest, der über ein Kohlenstoffatom des Ringes mit dem Grundkörper verknüpft ist und der zusätzlich an jedem weiteren Kohlenstoffatom noch einen der folgenden Substituenten tragen kann:

- Cyano, Thiocyanato, Nitro;
- Halogen, insbesondere Fluor, Chlor und Brom;
- $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl und Ethyl;
- partiell oder vollständig halogeniertes $C_1$-$C_4$-Alkyl,bevorzugt $C_1$-$C_2$-Alkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
- $C_3$-$C_{10}$-Cycloalkyl, das im Falle der großen Ringe bis zu tricyclisch sein und das jeweils ein- oder zweifach durch Methyl substituiert sein kann, insbesondere Cyclopropyl, Cyclobutyl, Cyclopentyl, Cyclohexyl, Adamantyl, 1-Methylcyclopropyl, 2-Methylcyclopropyl, 2,2-Dimethylcyclopropyl, 1-Methylcyclopentyl und 1-Methylcyclohexyl;

- $C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy und Ethoxy;
- $C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
- Phenyl, Naphthyl, Benzyl oder Thienyl, wobei der Aromat jeweils bis zu drei Halogenatome, bevorzugt Fluor und Chlor und/oder $C_1$-$C_4$-Alkylgruppen, bevorzugt Methyl und Ethyl, tragen kann, insbesondere Phenyl, Naphth-1-yl, 4-Chlor-naphth-1-yl, Thien-2-yl und 5-Chlor-thien-2-yl;
- CO-$R^5$, CO-O$R^5$, N$R^6R^7$, wobei $R^6$ und $R^7$ $C_1$-$C_4$-Alkyl, insbesondere Methyl und Ethyl, $C_2$-$C_4$-Alkenyl, insbesondere Ethenyl, Allyl und 2-Butenyl, oder $C_2$-$C_4$-Alkinyl, insbesondere Ethinyl und 2-Propinyl, bedeutet;

oder die Substituenten an zwei benachbarten Kohlenstoffatomen bilden eine $C_3$-$C_6$-Methylenkette.

Besonders geeignete Heterocyclen sind Thien-2-yl, Thien-3-yl, 5-Chlorthien-2-yl, 5-Bromthien-2-yl, 5-Nitro-thien-2-yl, 5-Cyano-thien-2-yl, 3-Cyano-thien-2-yl, 4-Chlor-3-cyano-thien-2-yl, 4-Brom-3-cyano-thien-2-yl, 4-Methyl-3-cyano-thien-2-yl, 4-Methyl-3,5-dicyano-thien-2-yl,4-Methyl-3,5-bis-(ethoxycarbonyl)-thien-2-yl, 4-Methyl-3,5-bis(methoxycarbonyl)-thien-2-yl, 4-Methyl-3-cyano-5-methoxycarbonyl-thien-2-yl, 4-Methyl-5-cyano-3-methoxycarbonyl-thien-2-yl, 4,5-Tetramethylen-3-ethoxycarbonyl-thien-2-yl, 4,5-Tetramethylen-3-methoxycarbonyl-thien-2-yl, 4,5-Trimethylen-3-methoxycarbonyl-thien-2-yl, 4,5-Tetramethylen-3-cyano-thien-2-yl, 4-Methoxycarbonyl-thien-3-yl, 4-Ethoxycarbonyl-thien-3-yl, 4-Cyano-thien-3-yl, 4-Nitro-thien-3-yl, 4,5-Dichlor-thien-3-yl, 2-Cyano-thien-3-yl, 2-Methoxycarbonyl-thien-3-yl, 2-Methoxycarbonyl-4-nitro-thien-3-yl, 2-Ethoxycarbonyl-4-nitro-thien-3-yl, 2-Ethoxycarbonyl-4-cyano-thien-3-yl, 2-Methoxycarbonyl-4-cyano-thien-3-yl, 2-Methoxycarbonyl-4-cyano-5-methyl-thien-3-yl, 2-Methoxycarbonyl-4-nitro-5-methyl-thien-3-yl, 2,4-Dicyano-thien-3-yl, 2,4-Dicyano-5-methyl-thien-3-yl, 2-Cyano-4-nitro-thien-3-yl, 2-Cyano-4-nitro-5-methyl-thien-3-yl, Isothiazol-5-yl, 3-Methyl-isothiazol-5-yl, 3-Methyl-4-thiocyanato-isothiazol-5-yl, 3-Methyl-4-brom-isothiazol-5-yl, 3-Methyl-4-chlor-isothiazol-5-yl, 3-Methyl-4-cyano-isothiazol-5-yl, 3-Methyl-4-nitro-isothiazol-5-yl, 3-Methyl-4-methoxycarbonyl-isothiazol-5-yl, 3-Phenyl-4-cyano-isothiazol-5-yl, 3-Phenyl-4-nitro-isothiazol-5-yl, 3-Phenyl-4-brom-isothiazol-5-yl, 3-Phenyl-4-chlor-isothiazol-5-yl, 3-Phenyl-4-methoxycarbonyl-isothiazol-5-yl, 3-Benzyl-4-cyano-isothiazol-5-yl, 3-Benzyl-4-nitro-isothiazol-5-yl, 3-Benzyl-4-brom-isothiazol-5-yl, 3-Benzyl-4-chlor-isothiazol-5-yl, 3-Benzyl-4-methoxycarbonyl-isothiazol-5-yl, Thiazol-2-yl, 5-Chlor-thiazol-2-yl, 5-Brom-thiazol-2-yl, 5-Methyl-thiazol-2-yl, 5-Methoxy-thiazol-2-yl, 5-Methylthio-thiazol-2-yl, 5-Ethoxy-thiazol-2-yl, 5-Ethylthio-thiazol-2-yl, 5-Cyano-thiazol-2-yl, 5-Methoxycarbonyl-thiazol-2-yl, 5-Ethoxycarbonyl-thiazol-2-yl, 5-Trifluormethyl-thiazol-2-yl, 5-(4-Chlorphenyl)-thiazol-2-yl, 5-(2,4-Dichlorphenyl)-thiazol-2-yl, 5-Benzyl-thiazol-2-yl, 4-Cyano-thiazol-2-yl, 4-Methoxycarbonyl-thiazol-2-yl, 4-Ethoxy-carbonyl-thiazol-2-yl, 4-Methyl-thiazol-2-yl, 4-Cyclohexyl-thiazol-2-yl, 4-Adamantyl-thiazol-2-yl, 4-Chlor-thiazol-2-yl, 4-Brom-thiazol-2-yl, 4-Nitro-thiazol-2-yl, 4-Trifluormethyl-thiazol-2-yl, 4-(2,4-Dichlorphenyl)-thiazol-2-yl, 4-(4-Fluorphenyl)-thiazol-2-yl, 4-(4-Chlorphenyl)-thiazol-2-yl, 4-(Naphth-1-yl)-thiazol-2-yl, 4-(4-Chlornaphth-1-yl)-thiazol-2-yl, 4-(Thien-2-yl)-thiazol-2-yl, 4-(5-Chlorthien-2-yl)-thiazol-2-yl, 4-Chlor-5-trifluormethyl-thiazol-2-yl, 4-Chlor-5-nitro-thiazol-2-yl, 4-Chlor-5-cyano-thiazol-2-yl, 4-Brom-5-cyano-thiazol-2-yl, 4-Brom-5-trifluormethyl-thiazol-2-yl, 4-Cyano-5-diallylamino-thiazol-2-yl, 4-Nitro-5-diallylamino-thiazol-2-yl, 1,3,4-Thiadiazol-2-yl, 5-Chlor-1,3,4-thiadiazol-2-yl, 5-Brom-1,3,4-thiadiazol-2-yl, 5-Methyl-1,3,4-thiadiazol-2-yl, 5-Nitro-1,3,4-thiadiazol-2-yl, 5-Cyano-1,3,4-thiadiazol-2-yl, 5-Methoxycarbonyl-1,3,4-thiadiazol-2-yl, 5-Methoxy-1,3,4-thiadiazol-2-yl, 5-Methylthio-1,3,4-thiadiazol-2-yl, 5-Trifluormethyl-1,3,4-thiadiazol-2-yl, 5-Benzyl-1,3,4-thiadiazol-2-yl, 5-Phenyl-1,3,4-thiadiazol-2-yl, 5-(4-Chlorphenyl)-1,3,4-thiadiazol-2-yl, 5-(4-Fluorphenyl)-1,3,4-thiadiazol-2-yl, 5-(2,4-Dichlorphenyl)-1,3,4-thiadiazol-2-yl, 1,2,4-Thiadiazol-5-yl, 3-Methyl-1,2,4-thiadiazol-5-yl, 3-Methylthio-1,2,4-thiadiazol-5-yl, 3-Methoxy-1,2,4-thiadiazol-5-yl, 3-Cyano-1,2,4-thiadiazol-5-yl, 3-Chlor-1,2,4-thiadiazol-5-yl, 3-Brom-1,2,4-thiadiazol-5-yl, 3-Trifluormethyl-1,2,4-thiadiazol-5-yl, 3-(4-Chlorphenyl)-1,2,4-thiadiazol-5-yl.

Die N-Heteroaryl-2-nitroaniline Ia sind besonders bevorzugt, wobei N-[3,5-Diethoxycarbonyl-4-methyl-thien-2-yl]-2,4-dinitro-6-trifluormethylanilin, N-[4-Brom-3-methyl-isothiazol-5-yl]-2,6-dinitro-4-trifluormethylanilin, N-(5-Chlorthiazol-2-yl)-2,6-dinitro-3-chlor-4-trifluormethylanilin und N-(5-Bromthiazol-2-yl)-2,6-dinitro-3-chlor-4-trifluormethylanilin ganz besonders bevorzugt sind.

Die N-Heteroaryl-2-nitroaniline Ia und Ib sind auf verschiedene Weise erhältlich, und zwar vorzugsweise nach den folgenden Methoden:

a) Umsetzung von hologenhaltigen Heteroaromaten mit 2-Nitroanilinen oder von Heteroarylaminen mit 2-Nitro-halogenbenzolen zu den Verbindungen Ia, wobei Q Wasserstoff bedeutet:

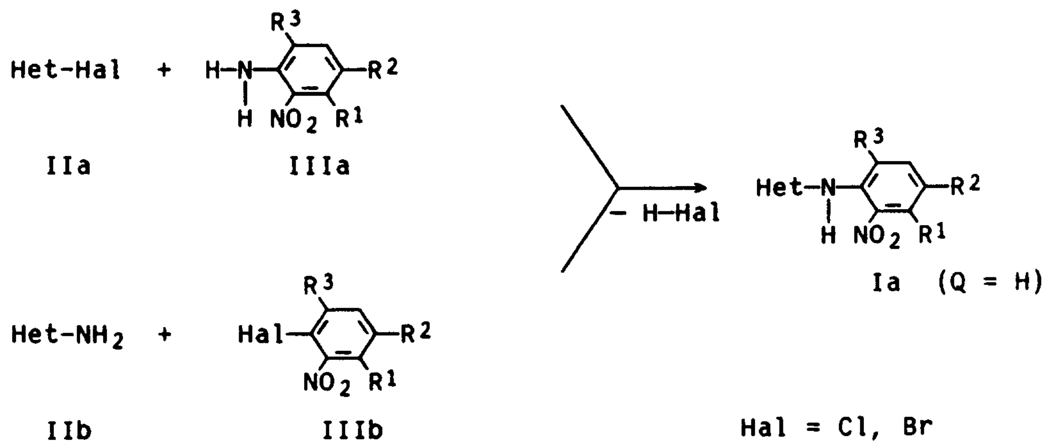


IIa IIIa

IIb IIIb

Ia (Q = H)

Hal = Cl, Br

Die Reaktion wird normalerweise in einem inerten Lösungs- bzw. Verdünnungsmittel, vorteilhaft in Anwesenheit einer Base, durchgeführt.

Geeignete Lösungsmittel sind aliphatische Kohlenwasserstoffe wie Cyclohexan und Petrolether, aromatische Kohlenwasserstoffe wie Toluol, o-, m-und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril, Propionitril und Dimethylformamid, Ketone wie Aceton, Methylethylketon und Diethylketon, Alkohole wie Methanol, Ethanol, n-Propanol, Isopropanol, n-Butanol und tert.-Butanol sowie Dimethylsulfoxid; besonders bevorzugt sind Tetrahydrofuran, Dioxan, Dimethylformamid und tert.-Butanol. Es können auch Gemische der genannten Lösungsmittel verwendet werden.

Bei oxidationsempfindlichen Reaktionspartnern empfiehlt sich das Arbeiten in einem wasserfreien Lösungsmittel unter einer Atmosphäre aus Inertgas wie Stickstoff, Helium und Argon, bevorzugt Stickstoff.

Als Basen eignen sich allgemein anorganische Verbindungen, beispielsweise Alkalimetall- und Erdalkalimetallhydroxide wie Lithiumhydroxid, Natriumhydroxid, Kaliumhydroxid, Calciumhydroxid, Alkalimetall- und Erdalkalimetalloxide wie Lithiumoxid, Natriumoxid, Calciumoxid und Magnesiumoxid, Alkalimetallhydride wie Lithiumhydrid und Natriumhydrid, Erdalkalimetallhydride wie Calciumhydrid, Alkalimetallamide wie Lithiumamid, Natriumamid und Kaliumamid, Alkalimetall- und Erdalkalimetallcarbonate wie Natriumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Lithiummethyl und Butyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-butanolat und Dimethoxymagnesium und organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine. Besonders bevorzugt werden Kaliumhydroxid, Natriumhydroxid, Kaliumcarbonat, Natriumethanolat und Kalium-tert.-butanolat sowie Triethylamin.

Vorteilhaft setzt man die Ausgangsverbindungen II und III in etwa stöchiometrischem Verhältnis ein, jedoch kann in manchen Fällen auch ein Überschuß der einen oder anderen Komponente, etwa bis zu 10 %, empfehlenswert sein.

Die Menge an Base ist nicht kritisch. Für eine vollständige Umsetzung wird in der Regel mindestens eine stöchiometrische Menge an Base, bezogen auf die Menge an II oder III, benötigt; bevorzugt verwendet man einen Überschuß an Base bis zu etwa 100 mol-%.

Im allgemeinen liegt die Reaktionstemperatur zwischen (-20)°C und dem Siedepunkt des jeweiligen Lösungsmittels, bevorzugt zwischen 0 und 50°C.

Da die Reaktion nicht druckabhängig ist, arbeitet man vorteilhaft bei Normaldruck oder unter dem Eigendruck des jeweiligen Lösungsmittels.

Die Ausgangsverbindungen IIa und IIIa sind bekannt oder können nach an sich bekannten Verfahren hergestellt werden.

b) Umsetzung von N-Heteroaryl-2-nitroanilinen Ia mit einem Acylierungs- oder Sulfonierungsmittel IV

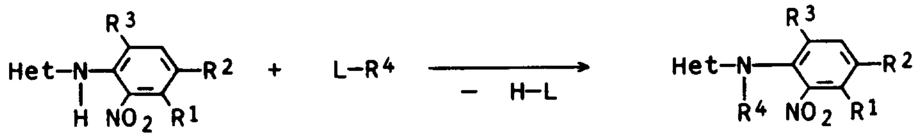

Ia IV Ib

wobei L eine nucleophile Abgangsgruppe, insbesondere einen Sulfonylrest wie Methylsulfonyl, Trifluormethylsulfonyl, p-Toluolsulfonyl, p-Bromphenylsulfonyl, einen Ethoxy- oder Phenoxyrest oder einen Carboxylatrest wie Acetat bedeutet, besonders bevorzugt Halogen wie Chlor, Brom und Jod.

Die Reaktion wird normalerweise in einem inerten Lösungs- bzw. Verdünnungsmittel, insbesondere in einem aprotischen Lösungsmittel wie u.a. für Methode a) genannt, durchgeführt.

Vorteilhaft setzt man alle Ausgangsverbindungen in etwa stöchiometrischem Verhältnis ein, jedoch kann in manchen Fällen auch ein Überschuß der einen oder anderen Komponente, etwa bis zu 10 mol-%, empfehlenswert sein.

Bezüglich der verwendbaren Basen sowie des Druckes gelten die Angaben für Methode a).

Im Allgemeinen liegt die Reaktionstemperatur zwischen (-20)°C und dem Siedepunkt des jeweiligen Lösungsmittels, insbesondere zwischen 20 und 150°C.

Die Acylierungs- und Sulfonierungsmittel IV sind bekannt oder können nach an sich bekannten Verfahren hergestellt werden.

Die Verbindungen Ia und Ib können nach den hierfür üblichen Methoden, z.B. durch Umkristallisation, Extraktion und Chromatographie, gereinigt werden.

Bei den N-Heteroaryl-2-nitroanilinen Ia kann das Proton am Stickstoffatom des Anilingrundkörpers leicht abgespalten werden. Die Verbindungen Ia weisen daher saure Eigenschaften auf; mit Basen bilden sie leicht basische Salze.

Die N-Heteroaryl-2-nitroaniline Ia' und Ib sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören:

- aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis;
- aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria;

- aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa;
- aus der Ordnung der Thripse (Thysanoptera) beispielsweise Frankliniella fusca, Frankliniella occidentalis, Frankliniella tritici, Scirtothrips citri, Thrips oryzae, Thrips palmi und Thrips tabaci;
- aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata und Solenopsis invicta;
- aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis und Thyanta perditor;
- aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae$_1$ Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii;
- aus der Ordnung der Termiten (Isoptera) beispielsweise Calotermes flavicollis, Leucotermes flavipes, Reticulitermes lucifugus und Termes natalensis;
- aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus;
- aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius und Tetranychus urticae;
- aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. eine innige Mischung aus 5 Gew.-Teilen der Verbindung Nr. 1.01 und 95 Gew.-Teilen feinteiligem Kaolin; dieses Stäubemittel enthält 5 Gew.% Wirkstoff;

II. eine innige Mischung aus 30 Gew.-Teilen der Verbindung Nr. 2.01, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit (Wirkstoffgehalt 23 Gew.%);

III. eine Lösung aus 10 Gew.-Teilen der Verbindung Nr. 3.01, 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl (Wirkstoffgehalt 9 Gew.%);

IV. eine Lösung aus 20 Gew.-Teilen der Verbindung Nr. 4.01, 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%);

V. eine in einer Hammermühle vermahlene Mischung aus 80 Gew.-Teilen der Verbindung Nr. 5.01, 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel (Wirkstoffgehalt 80 Gew.%); durch feines Verteilen der Mischung in Wasser erhält man eine Spritzbrühe;

VI. eine Lösung aus 90 Gew.-Teilen der Verbindung Nr. 6.01 und 10 Gew.-Teilen N-Methyl-$\alpha$-pyrrolidon, die zur Anwendung in Form kleinster Tropfen geeignet ist (Wirkstoffgehalt 90 Gew.%);

VII. eine Lösung aus 20 Gew.-Teilen der Verbindung Nr. 2.02, 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Lösung in 100 000 Gew.-Teilen Wasser erhält man eine Dispersion, die 0,02 Gew.% des Wirkstoffs enthält;

VIII. eine in einer Hammermühle vermahlene Mischung aus 20 Gew.-Teilen der Verbindung Nr. 3.02, 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel; durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Mittel enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,02 bis 10, vorzugsweise 0,1 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiel

N-[4-(2,4-Dichlorphenyl)-thiazol-2-yl]-2,4-dinitro-6-trifluormethylanilin

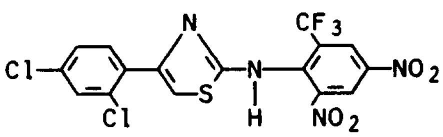

Zu einer Mischung aus 8,0 g (33 mmol) 2-Amino-4-(2,4-dichlorphenyl)thiazol,9,8 g (37 mmol) 2,4-Dinitro-6-trifluormethyl-chlorbenzol und 100 ml Tetrahydrofuran/tert.-Butanol (1:2) wurde unter starkem Rühren bei 0 bis 5 °C eine Lösung von 7,4 g (66 mmol) Kalium-tert.-butylat in 50 ml tert.-Butanol so zugetropft, daß die Zugabe nach 1 Stunde beendet war. Nach einer weiteren Stunde bei 0 °C wurde langsam, etwa im Verlauf von 2 Stunden, auf 20 °C erwärmt. Danach brachte man die Mischung mit Eisessig auf einen pH-Wert von 4 und verdünnte mit ca. 1 l Wasser. Der gebildete Niederschlag wurde abgetrennt, mit Wasser neutral gewaschen und getrocknet. Ausbeute: 90 %; Fp.: 125 bis 130 °C.

In der folgenden Tabelle sind noch weitere Verbindungen Ia aufgeführt, die auf die gleiche Weise hergestellt wurden oder herstellbar sind.

Tabelle

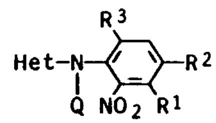

Ia

| Nr. | Het | Q | $R^1$ | $R^2$ | $R^3$ | Fp. [°C] |
|---|---|---|---|---|---|---|
| 1.01 | 3-CN-4-$CH_3$-5-$COOCH_3$-thien-2-yl | H | Cl | $CF_3$ | $NO_2$ | 189-190 |
| 1.02 | 3-CN-4-$CH_3$-5-$COOCH_3$-thien-2-yl | H | H | $CF_3$ | $NO_2$ | 202-204 |
| 1.03 | 3-CN-4-$CH_3$-5-$COOCH_3$-thien-2-yl | H | H | $NO_2$ | $CF_3$ | 164-165 |
| 1.04 | 4-$CH_3$-3,5-$(COOC_2H_5)_2$-thien-2-yl | H | Cl | $CF_3$ | $NO_2$ | 121-123 |
| 1.05 | 4-$CH_3$-3,5-$(COOC_2H_5)_2$-thien-2-yl | H | H | $CF_3$ | $NO_2$ | 178-179 |
| 1.06 | 4-$CH_3$-3,5-$(COOC_2H_5)_2$-thien-2-yl | H | H | $NO_2$ | $CF_3$ | 151-152 |
| 1.07 | 3-$COOC_2H_5$-4,5$(CH_2)_4$-thien-2-yl | H | Cl | $CF_3$ | $NO_2$ | 115-116 |
| 1.08 | 3-$COOC_2H_5$-4,5$(CH_2)_4$-thien-2-yl | H | H | $CF_3$ | $NO_2$ | 131-134 |
| 1.09 | 3-$COOC_2H_5$-4,5$(CH_2)_4$-thien-2-yl | H | H | $NO_2$ | $CF_3$ | 152-153 |
| 1.10 | 3-CN-4-Cl-thien-2-yl | H | H | $NO_2$ | $CF_3$ | öl |
| 2.01 | 4-$COOCH_3$-thien-3-yl | H | H | $CF_3$ | $NO_2$ | 208-209 |
| 2.02 | 2-$COOCH_3$-4-$NO_2$-thien-3-yl | H | Cl | $CF_3$ | $NO_2$ | 170-171 |
| 2.03 | 2-$COOCH_3$-4-$NO_2$-thien-3-yl | H | H | $CF_3$ | $NO_2$ | 184-185 |
| 2.04 | 2-$COOCH_3$-4-$NO_2$-thien-3-yl | H | H | $NO_2$ | $CF_3$ | 141-143 |
| 2.05 | 2-$COOCH_3$-4-CN-5-$CH_3$-thien-3-yl | H | Cl | $CF_3$ | $NO_2$ | 122-125 |
| 2.06 | 2-$COOCH_3$-4-CN-5-$CH_3$-thien-3-yl | H | H | $CF_3$ | $NO_2$ | 166-168 |
| 2.07 | 2-$COOCH_3$-4-CN-5-$CH_3$-thien-3-yl | H | H | $NO_2$ | $CF_3$ | 130-134 |
| 3.01 | 3-$CH_3$-isothiazol-5-yl | H | Cl | $CF_3$ | $NO_2$ | 90- 93 |
| 3.02 | 3-$CH_3$-isothiazol-5-yl | H | H | $CF_3$ | $NO_2$ | 111-114 |

Tabelle (Fortsetzung)

| Nr. | Het | Q | $R^1$ | $R^2$ | $R^3$ | Fp. [°C] |
|---|---|---|---|---|---|---|
| 3.03 | 3-$CH_3$-isothiazol-5-yl | H | H | $NO_2$ | $CF_3$ | 111-116 |
| 3.04 | 3-$CH_3$-4-SCN-isothiazol-5-yl | H | Cl | $CF_3$ | $NO_2$ | 126-128 |
| 3.05 | 3-$CH_3$-4-SCN-isothiazol-5-yl | H | H | $CF_3$ | $NO_2$ | 176-177 |
| 3.06 | 3-$CH_3$-4-Br-isothiazol-5-yl | H | Cl | $CF_3$ | $NO_2$ | 157-158 |
| 3.07 | 3-$CH_3$-4-Br-isothiazol-5-yl | H | H | $CF_3$ | $NO_2$ | 174-175 |
| 3.08 | 3-$CH_3$-4-Br-isothiazol-5-yl | H | H | $NO_2$ | $CF_3$ | 90- 92 |
| 3.09 | 3-$C_6H_5$-4-CN-isothiazol-5-yl | H | Cl | $CF_3$ | $NO_2$ | 134-136 |
| 3.10 | 3-$C_6H_5$-4-CN-isothiazol-5-yl | H | H | $CF_3$ | $NO_2$ | 144-147 |
| 3.11 | 3-$C_6H_5$-4-CN-isothiazol-5-yl | H | H | $NO_2$ | $CF_3$ | 193-196 |
| 3.12 | 3-$CH_2C_6H_5$-4-CN-isothiazol-5-yl | H | Cl | $CF_3$ | $NO_2$ | 149-152 |
| 3.13 | 3-$CH_2C_6H_5$-4-CN-isothiazol-5-yl | H | H | $CF_3$ | $NO_2$ | 178-180 |
| 3.14 | 3-$CH_2C_6H_5$-4-CN-isothiazol-5-yl | H | H | $NO_2$ | $CF_3$ | 135-138 |
| 4.01 | Thiazol-2-yl | H | H | $NO_2$ | $CF_3$ | 197-200 |
| 4.02 | 5-Cl-thiazol-2-yl | H | Cl | $CF_3$ | $NO_2$ | 152-162 |
| 4.03 | 5-Cl-thiazol-2-yl | H | H | $CF_3$ | $NO_2$ | 132-134 |
| 4.04 | 5-Cl-thiazol-2-yl | H | H | $NO_2$ | $CF_3$ | 125-130 |
| 4.05 | 5-Br-thiazol-2-yl | H | Cl | $CF_3$ | $NO_2$ | 141-144 |
| 4.06 | 5-Br-thiazol-2-yl | H | H | $CF_3$ | $NO_2$ | 142-145 |
| 4.07 | 5-Br-thiazol-2-yl | H | H | CN | $NO_2$ | 156-158 |
| 4.08 | 5-Br-thiazol-2-yl | H | H | $NO_2$ | $CF_3$ | 64- 67 |
| 4.09 | 5-$CH_3$-thiazol-2-yl | H | Cl | $CF_3$ | $NO_2$ | > 220 |
| 4.10 | 5-$CH_3$-thiazol-2-yl | H | H | $CF_3$ | $NO_2$ | 122-130 |
| 4.11 | 5-$CH_3$-thiazol-2-yl | H | H | $NO_2$ | $CF_3$ | 168-171 |

Tabelle (Fortsetzung)

| Nr. | Het | Q | $R^1$ | $R^2$ | $R^3$ | Fp. [°C] |
|---|---|---|---|---|---|---|
| 4.12 | 5-$SCH_3$-thiazol-2-yl | H | Cl | $CF_3$ | $NO_2$ | 145-148 |
| 4.13 | 5-$SCH_3$-thiazol-2-yl | H | H | $CF_3$ | $NO_2$ | 122 |
| 4.14 | 5-$SCH_3$-thiazol-2-yl | H | H | $NO_2$ | $CF_3$ | 143-144 |
| 4.15 | 5-$COOCH_3$-thiazol-2-yl | H | Cl | $CF_3$ | $NO_2$ | 85- 91 |
| 4.16 | 5-$COOCH_3$-thiazol-2-yl | H | H | $CF_3$ | $NO_2$ | 145-149 |
| 4.17 | 5-$COOCH_3$-thiazol-2-yl | H | H | $NO_2$ | $CF_3$ | 160-162 |
| 4.18 | 4-$COOC_2H_5$-thiazol-2-yl | H | Cl | $CF_3$ | $NO_2$ | 150 |
| 4.19 | 4-$COOC_2H_5$-thiazol-2-yl | H | H | $CF_3$ | $NO_2$ | 126-129 |
| 4.20 | 4-$COOC_2H_5$-thiazol-2-yl | H | H | $NO_2$ | $CF_3$ | 123-124 |
| 4.21 | 4-Adamantyl-thiazol-2-yl | H | Cl | $CF_3$ | $NO_2$ | 197-198 |
| 4.22 | 4-Adamantyl-thiazol-2-yl | H | H | $CF_3$ | $NO_2$ | 147-148 |
| 4.23 | 4-Adamantyl-thiazol-2-yl | H | H | $NO_2$ | $CF_3$ | 133-134 |
| 4.24 | 4-(2,4-$Cl_2$-$C_6H_3$)-thiazol-2-yl | H | Cl | $CF_3$ | $NO_2$ | 203-208 |
| 4.25 | 4-(2,4-$Cl_2$-$C_6H_3$)-thiazol-2-yl | H | H | $CF_3$ | $NO_2$ | 148-157 |
| 4.26 | 4-(2,4-$Cl_2$-$C_6H_3$)-thiazol-2-yl | H | H | $NO_2$ | $CF_3$ | 191-194 |
| 4.27 | 4-(4-F-$C_6H_4$)-thiazol-2-yl | H | Cl | $CF_3$ | $NO_2$ | 175-176 |
| 4.28 | 4-(4-F-$C_6H_4$)-thiazol-2-yl | H | H | $CF_3$ | $NO_2$ | 177-179 |
| 4.29 | 4-(4-F-$C_6H_4$)-thiazol-2-yl | H | H | $NO_2$ | $CF_3$ | 158-160 |
| 4.30 | 4-(4-Cl-Naphth-1-yl)-thiazol-2-yl | H | Cl | $CF_3$ | $NO_2$ | > 220 |
| 4.31 | 4-(4-Cl-Naphth-1-yl)-thiazol-2-yl | H | H | $NO_2$ | $CF_3$ | > 220 |
| 4.32 | 4-(5-Cl-Thien-2-yl)-thiazol-2-yl | H | H | $CF_3$ | $NO_2$ | 176-178 |
| 4.33 | 4-(Thien-2-yl)-thiazol-2-yl | H | Cl | $CF_3$ | $NO_2$ | 148-151 |
| 4.34 | 4-Cl-5-$CF_3$-thiazol-2-yl | H | Cl | $CF_3$ | $NO_2$ | Öl |

Tabelle (Fortsetzung)

| Nr. | Het | Q | $R^1$ | $R^2$ | $R^3$ | Fp. [°C] |
|---|---|---|---|---|---|---|
| 4.35 | 4-CN-5-N(allyl)$_2$-thiazol-2-yl | H | Cl | $CF_3$ | $NO_2$ | 125-130 |
| 5.01 | 5-Br-(1,3,4-thiadiazol-2-yl) | H | Cl | $CF_3$ | $NO_2$ | 211-213 |
| 5.02 | 5-Br-(1,3,4-thiadiazol-2-yl) | H | H | $CF_3$ | $NO_2$ | 217-218 |
| 5.03 | 5-Br-(1,3,4-thiadiazol-2-yl) | H | H | $NO_2$ | $CF_3$ | 168-169 |
| 5.04 | 5-$CH_3$-(1,3,4-thiadiazol-2-yl) | H | Cl | $CF_3$ | $NO_2$ | 131-134 |
| 5.05 | 5-$CH_3$-(1,3,4-thiadiazol-2-yl) | H | $OCH_3$ | $CF_3$ | $NO_2$ | 128-132 |
| 5.06 | 5-$CH_3$-(1,3,4-thiadiazol-2-yl) | H | H | $NO_2$ | Cl | 195-197 |
| 5.07 | 5-$CH_3$-(1,3,4-thiadiazol-2-yl) | H | H | $NO_2$ | $CF_3$ | 162-164 |
| 5.08 | 5-(4-Cl-$C_6H_4$)-(1,3,4-thiadiazol-2-yl) | H | Cl | $CF_3$ | $NO_2$ | 193-196 |
| 5.09 | 5-(4-Cl-$C_6H_4$)-(1,3,4-thiadiazol-2-yl) | H | H | $CF_3$ | $NO_2$ | 210-214 |
| 5.10 | 5-(4-Cl-$C_6H_4$)-(1,3,4-thiadiazol-2-yl) | H | H | $NO_2$ | $CF_3$ | 151-154 |
| 5.11 | 5-$CF_3$-(1,3,4-thiadiazol-2-yl) | H | Cl | $CF_3$ | $NO_2$ | 177-181 |
| 5.12 | 5-$CF_3$-(1,3,4-thiadiazol-2-yl) | H | H | $CF_3$ | $NO_2$ | 195-196 |
| 5.13 | 5-$CF_3$-(1,3,4-thiadiazol-2-yl) | H | H | $NO_2$ | $CF_3$ | 176-183 |
| 5.14 | 5-$CF_3$-(1,3,4-thiadiazol-2-yl) | H | $OCH_3$ | $CF_3$ | $NO_2$ | 158-160 |
| 5.15 | 5-$CF_3$-(1,3,4-thiadiazol-2-yl) | H | H | CN | $NO_2$ | > 220 |
| 5.16 | 5-$CF_3$-(1,3,4-thiadiazol-2-yl) | H | H | $NO_2$ | Cl | 191-193 |
| 5.17 | 5-$CF_3$-(1,3,4-thiadiazol-2-yl) | H | H | $NO_2$ | $CHCH_3C_2H_5$ | 192-194 |
| 6.01 | 3-$SCH_3$-(1,2,4-thiadiazol-5-yl) | H | Cl | $CF_3$ | $NO_2$ | 112-116 |
| 6.02 | 3-$SCH_3$-(1,2,4-thiadiazol-5-yl) | H | H | $CF_3$ | $NO_2$ | 146-148 |
| 6.03 | 3-$SCH_3$-(1,2,4-thiadiazol-5-yl) | H | H | $NO_2$ | $CF_3$ | Harz |
| 7.01 | 5-Cl-thiazol-2-yl | $N(n-C_4H_9)_4$ | Cl | $CF_3$ | $NO_2$ | 64-66 |
| 7.02 | 4-(2,4-$Cl_2$-$C_6H_3$)thiazol-2-yl | $N(n-C_4H_9)_4$ | H | $NO_2$ | $CF_3$ | 90-96 |

Anwendungsbeispiele

Die insektizide Wirkung der Verbindungen der allgemeinen Formel Ia und Ib ließ sich durch folgende Versuche zeigen:

Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

Versuch A

Caenorhabditis elegans (Freilebende Nematoden), Kontaktwirkung

Der Boden eines Versuchsgefäßes wurde mit der acetonischen Lösung des Wirkstoffs benetzt und nach dem Abdampfen des Lösungsmittels mit 30 µl E.-Coli-Bakteriensuspension als Nährmedium bedeckt und mit 50 µl Nematodensuspension infiziert.

Nach 48 h wurde die Mortalitätsrate bestimmt.

In diesem Test zeigten die Verbindungen 1.1, 1.6, 1.9, 2.4, 2.7, 3.1, 3.3, 4.2, 4.5, 4.12, 4.14, 4.17, 4.27, 4.29, 4.31, 4.32, 4.34, 5.1, 5.3, 5.8, 5.10, 5.12, 6.1, 6.2 und 6.3 Wirkschwellen von 0,4 bis 100 ppm.

Versuch B

Tetranychus telarius (Rote Spinne), Kontaktwirkung

In Töpfen befindliche Buschbohnen, die das zweite Folgeblattpaar gebildet hatten und die stark mit den Spinnmilben befallen waren, wurden mit verschieden konzentrierten wässrigen Wirkstoffaufbereitungen tropfnaß gespritzt. Dazu besprühte man die Pflanzen auf einem Drehteller von allen Seiten mit ca. 50 ml der Spritzbrühe.

Nach 5 Tagen im Gewächshaus wurde der Bekämpfungserfolg mittels Mikroskop (Binokular) bestimmt.

In diesem Test zeigten die Verbindungen 3.3, 3.8, 4.14, 4.17, 4.26 und 5.7 Wirkschwellen von 200 bis 1000 ppm.

Versuch C

Plutella maculipennis (Kohlschaben - Raupe), Kontaktwirkung

Blätter junger Kohlpflanzen wurden mit der wässrigen Wirkstoffaufbereitung benetzt und anschließend auf einen angefeuchteten Filter gelegt. Die präparierten Blätter wurden anschließend mit jeweils 10 Raupen des 4. Entwicklungsstadiums belegt.

Nach 48 h wurde die Mortalitätsrate bestimmt.

In diesem Test zeigten die Verbindungen 3.3, 3.7, 3.8, 4.11, 4.25, 4.26, 4.29, 4.32, 5.3, 5.7, 5.10, 5.15, 5.17, 6.2 und 6.3 Wirkschwellen von 40 bis 1000 ppm.

**Patentansprüche**

**Patentansprüche für folgende Vertragsstaaten : BE, CH, DE, FR, GB, IT, LI, NL**

**1.** N-Heteroaryl-2-nitroaniline der allgemeinen Formel Ia und Ib,

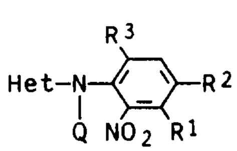

Ia

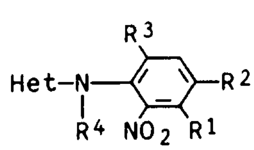

Ib

in der die Variablen die folgende Bedeutung haben:

$R^1$ Wasserstoff, Halogen oder $C_1$-$C_4$-Alkoxy;

$R^2$ Nitro, Cyano, Halogen, partiell oder vollständig halogeniertes $C_1$-$C_4$-Alkyl;
$R^3$ Nitro, Halogen, $C_1$-$C_6$-Alkyl, partiell oder vollständig halogeniertes $C_1$-$C_4$-Alkyl;
$R^4$ -CO-$R^5$, -CO-$OR^5$ oder -$SO_2R_5$ mit
$R^5$ $C_1$-$C_4$-Alkyl, Phenyl oder Naphthyl, die beide noch bis zu 3 Halogenatome und/oder $C_1$-$C_4$-Alkylgruppen tragen können;
Q Wasserstoff, ein Alkalimetall- oder Erdalkalimetallion, ein Ammoniumion, dessen Stickstoffatom bis zu vier $C_1$-$C_4$-Alkyl-, Hydroxy-$C_1$-$C_4$-alkyl-, Phenyl- und/oder Benzylsubstituenten tragen kann, ein Phosphonium-, Sulfonium- oder Sulfoxoniumion oder ein Äquivalent eines Übergangsmetallkations;
Het einen Thienyl-, Thiazolyl-, Isothiazolyl- oder Thiadiazolyl-Rest, der über ein Kohlenstoffatom des Ringes mit dem Grundkörper verknüpft ist, an den ein nichtaromatischer $C_5$-$C_8$-Ring annelliert sein kann und dessen Grundkörper zusätzlich an jedem weiteren Kohlenstoffatom noch einen der folgenden Substituenten tragen kann:
Cyano, Thiocyanato, Nitro, Halogen, $C_1$-$C_4$-Alkyl, partiell oder vollständig halogeniertes $C_1$-$C_4$-Alkyl, $C_3$-$C_{10}$-Cycloalkyl, $C_1$-$C_4$-Alkoxy, $C_1$-$C_4$-Alkylthio, Phenyl, Naphthyl, Benzyl oder Thienyl, wobei der Aromat jeweils bis zu drei Halogenatome und/oder $C_1$-$C_4$-Alkylgruppen tragen kann; CO-$R^5$, CO-$OR^5$, $NR^6R^7$, wobei $R^6$ und $R^7$ $C_1$-$C_4$-Alkyl, $C_2$-$C_4$-Alkenyl oder $C_2$-$C_4$-Alkinyl bedeuten,

ausgenommen diejenigen Verbindungen Ia, bei denen $R^1$ Wasserstoff, $R^2$ und/oder $R^3$ Nitro und Het einen durch Trifluormethyl oder tert.-Butyl substituierten 1,3,4-Thiadiazol-2-yl-Rest bedeuten.

**2.** N-Heteroaryl-2-nitroaniline der allgemeinen Formel Ia gemäß Anspruch 1.

**3.** Verfahren zur Herstellung der N-Heteroaryl-2-nitroaniline Ia gemäß Anspruch 1, dadurch gekennzeichnet, daß man entweder einen halogensubstituierten Heteroaromat der Formel IIa

Het-Hal IIa

wobei Hal Chlor oder Brom bedeutet,
mit einem 2-Nitroanilin der Formel IIIa

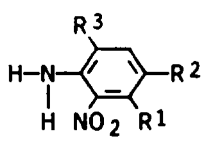

IIIa

oder ein Heteroaryl-amin der Formel IIb

Het-$NH_2$ IIb

mit einem 2-Nitro-1-halogenbenzol der Formel III b

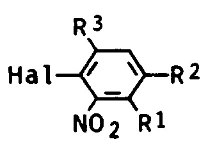

IIIb,

gewünschtenfalls in Anwesenheit einer Base, umsetzt, und die Verfahrensprodukte Ia mit Q = Wasserstoff in ihre basischen Salze überführt.

**4.** Verfahren zur Herstellung der N-Heteroaryl-2-nitroaniline Ib gemäß Anspruch 1, dadurch gekennzeichnet, daß man ein N-Heteroaryl-2-nitroanilin der Formel Ia mit Q = Wasserstoff, gewünschtenfalls in Gegenwart einer Base, mit einem Acylierungs- oder Sulfonierungsmittel der Formel IV

L-$R^4$ IV

wobei L eine nukleophil substituierbare Abgangsgruppe bedeutet, umsetzt.

5. Mittel zur Bekämpfung von Insekten, Spinnentieren und Nematoden, enthaltend inerte Trägerstoffe und mindestens eine pestizid wirksame Menge eines N-Heteroaryl-2-nitroanilins der Formal Ia' oder Ib, wobei Ia' der Definition von Ia ohne die Ausnahmebestimmung entspricht.

6. Mittel zur Bekämpfung von Insekten, Spinnentieren und Nematoden nach Anspruch 5, enthaltend 0,1 bis 99 Gew.-% eines N-Heteroaryl-2-nitroanilins Ia' und/oder Ib.

7. Verwendung der N-Heteroaryl-2-nitroaniline Ia' bzw. Ib gemäß Anspruch 5 oder 6 zur Bekämpfung von Insekten, Spinnentieren und Nematoden.

8. Verfahren zur Bekämpfung von Insekten, Spinnentieren und Nematoden, dadurch gekennzeichnet, daß man eine pestizid wirksame Menge eines N-Heteroaryl-2-nitroanilins der Formel Ia' und/oder Ib gemäß Anspruch 5 oder 6 auf Insekten, Spinnentiere und Nematoden und/oder ihren Lebensraum einwirken läßt.

**Patentansprüche für folgenden Vertragsstaat : ES**

1. Mittel zur Bekämpfung von Insekten, Spinnentieren und Nematoden, enthaltend inerte Trägerstoffe und mindestens eine pestizid wirksame Menge eines N-Heteroaryl-2-nitroanilins der allgemeinen Formel Ia und Ib,

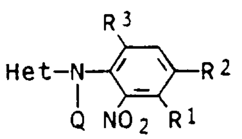

Ia

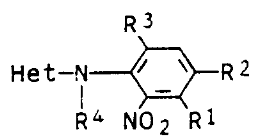

Ib

in der die Variablen die folgende Bedeutung haben:

- $R^1$ Wasserstoff, Halogen oder $C_1$-$C_4$-Alkoxy;
- $R^2$ Nitro, Cyano, Halogen, partiell oder vollständig halogeniertes $C_1$-$C_4$-Alkyl;
- $R^3$ Nitro, Halogen, $C_1$-$C_6$-Alkyl, partiell oder vollständig halogeniertes $C_1$-$C_4$-Alkyl;
- $R^4$ -CO-$R^5$, -CO-O$R^5$ oder -$SO_2R_5$ mit
- $R^5$ $C_1$-$C_4$-Alkyl, Phenyl oder Naphthyl, die beide noch bis zu 3 Halogenatome und/oder $C_1$-$C_4$-Alkylgruppen tragen können;
- Q Wasserstoff, ein Alkalimetall- oder Erdalkalimetallion, ein Ammoniumion, dessen Stickstoffatom bis zu vier $C_1$-$C_4$-Alkyl-, Hydroxy-$C_1$-$C_4$-alkyl-, Phenyl- und/oder Benzylsubstituenten tragen kann, ein Phosphonium-, Sulfonium- oder Sulfoxoniumion oder ein Äquivalent eines Übergangsmetallkations;
- Het einen Thienyl-, Thiazolyl-, Isothiazolyl- oder Thiadiazolyl-Rest, der über ein Kohlenstoffatom des Ringes mit dem Grundkörper verknüpft ist, an den ein nichtaromatischer $C_5$-$C_8$-Ring annelliert sein kann und dessen Grundkörper zusätzlich an jedem weiteren Kohlenstoffatom noch einen der folgenden Substituenten tragen kann:
  Cyano, Thiocyanato, Nitro, Halogen, $C_1$-$C_4$-Alkyl, partiell oder vollständig halogeniertes $C_1$-$C_4$-Alkyl, $C_3$-$C_{10}$-Cycloalkyl, $C_1$-$C_4$-Alkoxy, $C_1$-$C_4$-Alkylthio, Phenyl, Naphthyl, Benzyl oder Thienyl, wobei der Aromat jeweils bis zu drei Halogenatome und/oder $C_1$-$C_4$-Alkylgruppen tragen kann; CO-$R^5$, CO-O$R^5$, $NR^6R^7$, wobei $R^6$ und $R^7$ $C_1$-$C_4$-Alkyl, $C_2$-$C_4$-Alkenyl oder $C_2$-$C_4$-Alkinyl bedeuten,

ausgenommen diejenigen Verbindungen Ia, bei denen $R^1$ Wasserstoff, $R^2$ und/oder $R^3$ Nitro und Het einen durch Trifluormethyl oder tert.-Butyl substituierten 1,3,4-Thiadiazol-2-yl-Rest bedeuten.

2. Verfahren zur Herstellung der N-Heteroaryl-2-nitroaniline Ia gemäß Anspruch 1, dadurch gekennzeichnet, daß man entweder einen halogensubstituierten Heteroaromat der Formel IIa

Het-Hal IIa

wobei Hal Chlor oder Brom bedeutet, mit einem 2-Nitroanilin der Formel IIIa

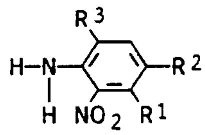

IIIa

oder ein Heteroaryl-amin der Formel IIb

Het-$NH_2$ IIb

mit einem 2-Nitro-1-halogenbenzol der Formel III b

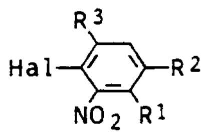

IIIb,

gewünschtenfalls in Anwesenheit einer Base, umsetzt, und die Verfahrensprodukte Ia mit Q = Wasserstoff in ihre basischen Salze überführt.

**3.** Verfahren zur Herstellung der N-Heteroaryl-2-nitroaniline Ib gemäß Anspruch 1, dadurch gekennzeichnet, daß man ein N-Heteroaryl-2-nitroanilin der Formel Ia mit Q = Wasserstoff, gewünschtenfalls in Gegenwart einer Base, mit einem Acylierungs- oder Sulfonierungsmittel der Formel IV

L-$R^4$ IV

wobei L eine nukleophil substituierbare Abgangsgruppe bedeutet, umsetzt.

**4.** Mittel zur Bekämpfung von Insekten, Spinnentieren und Nematoden nach Anspruch 1, enthaltend 0,1 bis 99 Gew.-% eines N-Heteroaryl-2-nitroanilins Ia und/oder Ib.

**5.** Verwendung der N-Heteroaryl-2-nitroaniline Ia', wobei Ia' der Definition von Ia ohne die Ausnahmebestimmungen entspricht, bzw. Ib gemäß Anspruch 1 zur Bekämpfung von Insekten, Spinnentieren und Nematoden.

**6.** Verfahren zur Bekämpfung von Insekten, Spinnentieren und Nematoden, dadurch gekennzeichnet, daß man eine pestizid wirksame Menge eines N-Heteroaryl-2-nitroanilins der Formel Ia' und/oder Ib gemäß Anspruch 1 oder 5 auf Insekten, Spinnentiere und Nematoden und/oder ihren Lebensraum einwirken laßt.

## Claims

**Claims for the following Contracting States : BE, CH, DE, FR, GB, IT, LI, NL**

**1.** An N-hetaryl-2-nitroaniline of the formula Ia or Ib

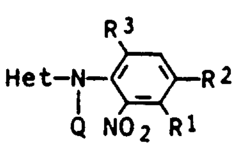

Ia

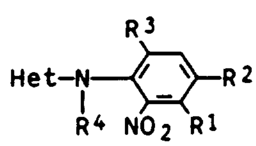

Ib

where

$R^1$ is hydrogen, halogen or $C_1$-$C_4$-alkoxy;
$R^2$ is nitro, cyano, halogen, partially or completely halogenated $C_1$-$C_4$-alkyl;
$R^3$ is nitro, halogen, $C_1$-$C_6$-alkyl, partially or completely halogenated $C_1$-$C_4$-alkyl;
$R^4$ is -CO-$R^5$, -CO-O$R^5$ or -$SO_2R_5$, with
$R^5$ being $C_1$-$C_4$-alkyl, phenyl or naphthyl, both of which can carry up to 3 halogen atoms and/or $C_1$-$C_4$-alkyl groups;
Q is hydrogen, an alkali metal or alkaline earth metal ion, an ammonium ion whose nitrogen can carry up to four $C_1$-$C_4$-alkyl, hydroxy-$C_1$-$C_4$-alkyl, phenyl and/or benzyl substituents, a phosphonium, sulfonium or sulfoxonium ion or an equivalent of a transition metal cation;
Het is thienyl, thiazolyl, isothiazolyl or thiadiazolyl, each of which is linked via a ring carbon to the basic element to which a non-aromatic $C_5$-$C_8$-ring can be fused and which can additionally carry on each other carbon one of the following substituents:
cyano, thiocyanato, nitro, halogen, $C_1$-$C_4$-alkyl, partially or completely halogenated $C_1$-$C_4$-alkyl, $C_3$-$C_{10}$-cycloalkyl, $C_1$-$C_4$-alkoxy, $C_1$-$C_4$-alkylthio, phenyl, naphthyl, benzyl or thienyl, where each aromatic ring can carry up to three halogen atoms and/or $C_1$-$C_4$-alkyl groups; CO-$R^5$, CO-O$R^5$, N$R^6R^7$, where $R^6$ and $R^7$ are $C_1$-$C_4$-alkyl, $C_2$-$C_4$-alkenyl or $C_2$-$C_4$-alkynyl,

excepting those compounds Ia where $R^1$ is hydrogen, $R^2$ and/or $R^3$ is nitro and Het is 1,3,4-thiadiazol-2-yl which is substituted by trifluoromethyl or tert-butyl.

**2.** An N-hetaryl-2-nitroaniline of the formula Ia as claimed in claim 1.

**3.** A process for preparing an N-hetaryl-2-nitroaniline Ia as claimed in claim 1, which comprises reacting either a halogen-substituted heteroaromatic compound of the formula IIa

Het-Hal IIa

where Hal is chlorine or bromine, with a 2-nitroaniline of the formula IIIa

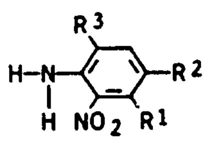

IIIa

or a hetarylamine of the formula IIb

Het-$NH_2$ IIb

with a 2-nitro-1-halobenzene of the formula IIIb

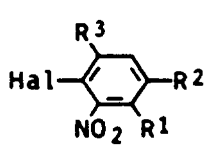

IIIb

if required in the presence of a base, and converting the product Ia with Q = hydrogen into a basic salt thereof.

**4.** A process for preparing an N-hetaryl-2-nitroaniline Ib as claimed in claim 1, which comprises reacting an N-hetaryl-2-nitroaniline of the formula Ia with Q = hydrogen, if required in the presence of a base, with an acylating or sulfonating agent of the formula IV

L-$R^4$ IV

where L is a leaving group amenable to nucleophilic substitution.

**5.** An agent for controlling insects, arachnids and nematodes, containing inert carriers and an N-hetaryl-2-nitroaniline of the formula Ia' or Ib, where Ia' corresponds to the definition of Ia without the specified exceptions, in an amount at least sufficient for pesticidal activity.

**6.** An agent for controlling insects, arachnids and nematodes as claimed in claim 5, containing from 0.1 to 99 % by weight of an N-hetaryl-2-nitroaniline Ia' and/or Ib.

**7.** The use of the N-hetaryl-2-nitroaniline Ia' or Ib as claimed in claim 5 or 6 for controlling insects, arachnids and nematodes.

**8.** A method for controlling insects, arachnids and nematodes, which comprises exposing insects, arachnids and nematodes and/or their habitat to an N-hetaryl-2-nitroaniline of the formula Ia' and/or Ib as claimed in claim 5 or 6 in an amount sufficient for pesticidal activity.

**Claims for the following Contracting State : ES**

**1.** An agent for controlling insects, arachnids and nematodes, containing inert carriers and an N-hetaryl-2-nitroaniline of the formula Ia or Ib

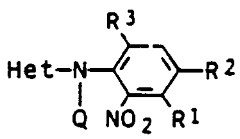

Ia

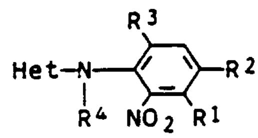

Ib

where

- $R^1$ is hydrogen, halogen or $C_1$-$C_4$-alkoxy;
- $R^2$ is nitro, cyano, halogen, partially or completely halogenated $C_1$-$C_4$-alkyl;
- $R^3$ is nitro, halogen, $C_1$-$C_6$-alkyl, partially or completely halogenated $C_1$-$C_4$-alkyl;
- $R^4$ is -CO-$R^5$, -CO-O$R^5$ or -$SO_2R_5$, with
- $R^5$ being $C_1$-$C_4$-alkyl, phenyl or naphthyl, both of which can carry up to 3 halogen atoms and/or $C_1$-$C_4$-alkyl groups;
- Q is hydrogen, an alkali metal or alkaline earth metal ion, an ammonium ion whose nitrogen can carry up to four $C_1$-$C_4$-alkyl, hydroxy-$C_1$-$C_4$-alkyl, phenyl and/or benzyl substituents, a phosphonium, sulfonium or sulfoxonium ion or an equivalent of a transition metal cation;
- Het is thienyl, thiazolyl, isothiazolyl or thiadiazolyl, each of which is linked via a ring carbon to the basic element to which a non-aromatic $C_5$-$C_8$-ring can be fused and which can additionally carry on each other carbon one of the following substituents:
  cyano, thiocyanato, nitro, halogen, $C_1$-$C_4$-alkyl, partially or completely halogenated $C_1$-$C_4$-alkyl, $C_3$-$C_{10}$-cycloalkyl, $C_1$-$C_4$-alkoxy, $C_1$-$C_4$-alkylthio, phenyl, naphthyl, benzyl or thienyl, where each aromatic ring can carry up to three halogen atoms and/or $C_1$-$C_4$-alkyl groups; CO-$R^5$, CO-O$R^5$, N$R^6R^7$, where $R^6$ and $R^7$ are $C_1$-$C_4$-alkyl, $C_2$-$C_4$-alkenyl or $C_2$-$C_4$-alkynyl,

excepting those compounds Ia where $R^1$ is hydrogen, $R^2$ and/or $R^3$ is nitro and Het is 1,3,4-thiadiazol-2-yl which is substituted by trifluoromethyl or tert-butyl, in an amount at least sufficient for pesticidal activity.

**2.** A process for preparing an N-hetaryl-2-nitroaniline Ia as claimed in claim 1, which comprises reacting either a halogen-substituted heteroaromatic compound of the formula IIa

Het-Hal IIa

where Hal is chlorine or bromine, with a 2-nitroaniline of the formula IIIa

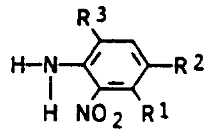

IIIa

or a hetarylamine of the formula IIb

Het-$NH_2$ IIb

with a 2-nitro-1-halobenzene of the formula IIIb

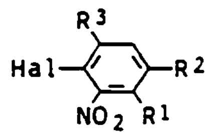

IIIb

if required in the presence of a base, and converting the product Ia with Q = hydrogen into a basic salt thereof.

**3.** A process for preparing an N-hetaryl-2-nitroaniline Ib as claimed in claim 1, which comprises reacting an N-hetaryl-2-nitroaniline of the formula Ia with Q = hydrogen, if required in the presence of a base, with an acylating or sulfonating agent of the formula IV

L-$R^4$ IV

where L is a leaving group amenable to nucleophilic substitution.

**4.** An agent for controlling insects, arachnids and nematodes as claimed in claim 1, containing from 0.1 to 99 % by weight of an N-hetaryl-2-nitroaniline Ia and/or Ib.

**5.** The use of the N-hetaryl-2-nitroaniline Ia', where Ia' corresponds to the definition of Ia without the specified exceptions, or Ib as claimed in claim 1 for controlling insects, arachnids and nematodes.

**6.** A method for controlling insects, arachnids and nematodes, which comprises exposing insects, arachnids and nematodes and/or their habitat to an N-hetaryl-2-nitroaniline of the formula Ia' and/or Ib as claimed in claim 1 or 5 in an amount sufficient for pesticidal activity.

**Revendications**

**Revendications pour les Etats contractants suivants : BE, CH, DE, FR, GB, IT, LI, NL**

**1.** N-hétéroaryle-2-nitroanilines de formule générale Ia ou Ib,

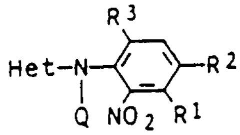

Ia

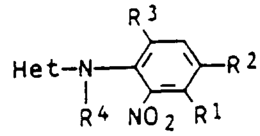

Ib

dans laquelle les variables ont les significations suivantes :

$R^1$ hydrogène, halogène ou alcoxy en C1-C4 ;

$R^2$ nitro, cyano, halogène, alkyle en C1 à C4 partiellement ou complètement halogéné ;
$R^3$ nitro, halogène, alkyle en C1 à C6, alkyle en C1 à C4 partiellement ou complètement halogéné ;
$R^4$ -CO-$R^5$, -CO-$OR^5$ ou -$SO_2R^5$,
$R^5$ représentant alkyle en C1 à C4, phényle ou naphtyle qui, tous deux, peuvent encore porter jusqu'à 3 atomes d'halogène et/ou groupes alkyle en C1 à C4 ;
Q hydrogène, un ion métal alcalin ou alcalino-terreux, un ion ammonium dont l'atome d'azote peut porter jusqu'à quatre substituants alkyle en C1 à C4, hydroxy-alkyle en C1 à C4, phényle et/ou benzyle, un ion phosphonium, sulfonium ou sulfoxonium ou un équivalent d'un cation de métal de transition ;
Het un reste thiényle, thiazolyle, isothiazolyle ou thiadiazolyle qui, par l'intermédiaire d'un atome de carbone du noyau, est relié au composé de base auquel peut être accolé un noyau en C5 à C8 non aromatique et dont le composé de base peut porter additionnellement, sur chaque autre atome de carbone, encore un des substituants suivants :
cyano, thiocyanato, nitro, halogène, alkyle en C1 à C4, alkyle en C1 à C4 partiellement ou complètement halogéné, cycloalkyle en C3 à C10, alcoxy en C1 à C4, alkylthio en C1 à C4, phényle, naphtyle, benzyle ou thiényle, l'aromatique pouvant porter dans chaque cas jusqu'à trois atomes d'halogène et/ou groupes alkyle en C1 à C4 ; CO-$R^5$, CO-$OR^5$, $NR^6R^7$, $R^6$ et $R^7$ représentant alkyle en C1 à C4, alcényle en C2 à C4 ou alcynyle en C2 à C4, les composés Ia dans lesquels $R^1$ représente hydrogène, $R^2$ et/ou $R^3$ représentant nitro et Het un reste 1,3,4-thiadiazol-2-yle substitué par trifluorométhyle ou ter-butyle, étant exceptés.

2. N-hétéroaryle-2-nitroanilines de formule générale Ia, selon la revendication 1.

3. Procédé de préparation des N-hétéroaryl-2-nitroanilines Ia selon la revendication 1, caractérisé par le fait que l'on fait réagir, éventuellement en présence d'une base, soit un hétéroaromatique substitué par halogène, de formule IIa

Het-Hal IIa

Hal représentant chlore ou brome,
avec une 2-nitroaniline de formule IIIa

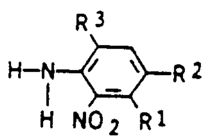

IIIa

ou une hétéroaryl-amine de formule IIb

HET-$NH_2$ IIb

avec un 2-nitro-1-halogénobenzène de formule IIIb

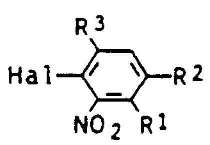

IIIb,

et on transforme les produits du procédé Ia, avec Q = hydrogène, en leurs sels basiques.

4. Procédé de préparation des N-hétéroaryl-2-nitroanilines Ib selon la revendication 1, caractérisé par le fait que l'on fait réagir, si souhaité en présence d'une base, une N-hétéroaryl-2-nitroaniline de formule Ia, avec Q = hydrogène, avec un agent d'acylation ou de sulfonation de formule IV

L-$R^4$ IV

L représentant un groupe substituable par un réactif nucléophile.

**5.** Agent de lutte contre les insectes, arachnides et nématodes, contenant des véhicules inertes et au moins une quantité efficace en tant que pesticide d'une N-hétéroaryl-2-nitroaniline de formule Ia' ou Ib, Ia' correspondant à la définition de Ia sans la disposition d'exception.

**6.** Agent de lutte contre les insectes, arachnides et nématodes, selon la revendication 5, contenant 0,1 à 99 % en poids d'une N-hétéroaryl-2-nitroaniline Ia' et/ou Ib.

**7.** Utilisation des N-hétéroaryl-2-nitroaniline Ia' ou Ib selon la revendication 5 ou 6 pour combattre des insectes, arachnides et nématodes.

**8.** Procédé de lutte contre les insectes, arachnides et nématodes, caractérisé par le fait que l'on fait agir une quantité efficace en tant que pesticide d'une N-hétéroaryl-2-nitroaniline de formule Ia' et/ou Ib, selon la revendication 5 ou 6, sur les insectes, arachnides et nématodes et/ou leur biotope.

**Revendications pour l'Etat contractant suivant : ES**

**1.** Agent de lutte contre des insectes, arachnides et nématodes, contenant des véhicules inertes et au moins une quantité efficace en tant que pesticide d'une N-hétéroaryl-2-nitroaniline de formule générale Ia ou Ib,

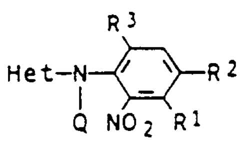

Ia

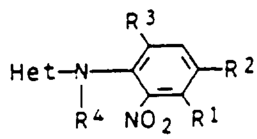

Ib

dans laquelle les variables ont les significations suivantes :
$R^1$ hydrogène, halogène ou alcoxy en C1-C4 ;
$R^2$ nitro, cyano, halogène, alkyle en C1 à C4 partiellement ou complètement halogéné ;
$R^3$ nitro, halogène, alkyle en C1 à C6, alkyle en C1 à C4 partiellement ou complètement halogéné ;
$R^4$ -CO-$R^5$, -CO-O$R^5$ ou -$SO_2R^5$,
$R^5$ représentant alkyle en C1 à C4, phényle ou naphtyle qui, tous deux, peuvent encore porter jusqu'à 3 atomes d'halogène et/ou groupes alkyle en C1 à C4 ;
Q hydrogène, un ion métal alcalin ou alcalino-terreux, un ion ammonium dont l'atome d'azote peut porter jusqu'à quatre substituants alkyle en C1 à C4, hydroxy-alkyle en C1 à C4, phényle et/ou benzyle, un ion phosphonium, sulfonium ou sulfoxonium ou un équivalent d'un cation de métal de transition ;
Het un reste thiényle, thiazolyle, isothiazolyle ou thiadiazolyle qui, par l'intermédiaire d'un atome de carbone du noyau, est relié au composé de base auquel peut être accolé un noyau en C5 à C8 non aromatique et dont le composé de base peut porter additionnellement, sur chaque autre atome de carbone, encore un des substituants suivants :
cyano, thiocyanato, nitro, halogène, alkyle en C1 à C4, alkyle en C1 à C4 partiellement ou complètement halogéné, cycloalkyle en C3 à C10, alcoxy en C1 à C4, alkylthio en C1 à C4, phényle, naphtyle, benzyle ou thiényle, l'aromatique pouvant porter dans chaque cas jusqu'à trois atomes d'halogène et/ou groupes alkyle en C1 à C4 ; CO-$R^5$, CO-O$R^5$, N$R^6R^7$, $R^6$ et $R^7$ représentant alkyle en C1 à C4, alcényle en C2 à C4 ou alcynyle en C2 à C4, les composés Ia dans lesquels $R^1$ représente hydrogène, $R^2$ et/ou $R^3$ représentant nitro et Het un reste 1,3,4-thiadiazol-2-yle substitué par trifluorométhyle ou ter-butyle, étant exceptés.

**2.** Procédé de préparation des N-hétéroaryl-2-nitroanilines Ia selon la revendication 1, caractérisé par le fait que l'on fait réagir, éventuellement en présence d'une base, soit un hétéroaromatique substitué par

halogène, de formule IIa

Het-Hal IIa

Hal représentant chlore ou brome,
avec une 2-nitroaniline de formule IIIa

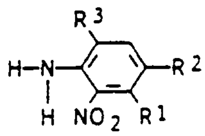

IIIa

ou une hétéroaryl-amine de formule IIb

HET-$NH_2$ IIb

avec un 2-nitro-1-halogénobenzène de formule IIIb

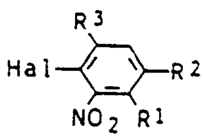

IIIb,

et on transforme les produits du procédé Ia, avec Q = hydrogène, en leurs sels basiques.

**3.** Procédé de préparation des N-hétéroaryl-2-nitroanilines Ib selon la revendication 1, caractérisé par le fait que l'on fait réagir, si souhaité en présence d'une base, une N-hétéroaryl-2-nitroaniline de formule Ia, avec Q = hydrogène, avec un agent d'acylation ou de sulfonation de formule IV

L-$R^4$ IV

L représentant un groupe substituable par un réactif nucléophile.

**4.** Agent de lutte contre des insectes, arachnides et nématodes, selon la revendication 1, contenant 0,1 à 99 % en poids d'une N-hétéroaryl-2-nitroaniline Ia et/ou Ib.

**5.** Utilisation des N-hétéroaryl-2-nitroanilines Ia', Ia' correspondant à la définition de Ia sans la disposition d'exception, ou de Ib, selon la revendication 1, pour la lutte contre des insectes, arachnides et nématodes.

**6.** Procédé de lutte contre des insectes, arachnides et nématodes, caractérisé par le fait que l'on fait réagir une quantité efficace en tant que pesticide d'une N-hétéroaryl-2-nitroaniline de formule Ia' et/ou Ib, selon la revendication 1 ou 5, sur des insectes, arachnides et nématodes et/ou leur biotope.